# EUROPEAN PATENT SPECIFICATION

(11) **EP 0 971 882 B1**
(45) Date of publication and mention of the grant of the patent: **04.12.2002**
(21) Application number: 98920498.7
(22) Date of filing: 24.03.1998
(51) Int. Cl.: C07C 233/78, A61K 31/165

(54) **NITRO-BENZAMIDE USEFUL AS ANTI-ARRHYTHMIC AGENT**
NITRO-BENZAMID VERWENDBAR ALS ANTIARRYTHMICA
NITRO-BENZAMIDE UTILE EN TANT QU'AGENT ANTI-ARYTHMIE

(30) Priority: 27.03.1997 GB 9706376
(43) Date of publication of application: 19.01.2000
(73) Proprietor: SmithKline Beecham plc, Brentford, Middlesex TW8 9GS (GB)
(72) Inventor: SLATER, Graham, Ralph, SmithKline Beecham Phar., Harlow, Essex CM19 5AW (GB); WESTLAKE, Paul, Jeffrey, SmithKline Beecham Phar., Harlow, Essex CM19 5AW (GB)
(74) Representative: Rutter, Keith, Dr.
(86) International application number: EP9801913
(87) International publication number: WO98043947

(56) References cited:
- WO-A-96/13479

## Description

This invention relates to a novel pharmaceutical, to a process for the preparation of the pharmaceutical and to the use of the pharmaceutical in medicine.

International Patent Application, Publication Number WO 96/13479 discloses certain compounds of formula (A): 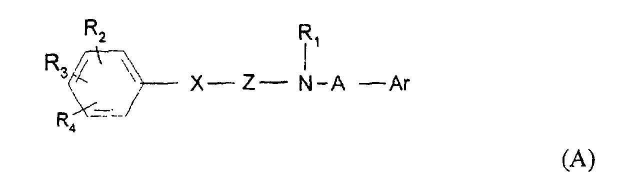 or a salt thereof, or a solvate thereof, characterised in that:
Ar represents substituted or unsubstituted aryl, wherein the optional substituents are selected from alkyl, hydroxy or alkoxy or, if attached to adjacent carbon atoms any two substituents together with the carbon atoms to which they are attached may form a fused heterocyclic ring of five to six atoms wherein one, two or three of the said atoms are oxygen or nitrogen;
A represents a C₁₋₄ n-alkylene group wherein each carbon is optionally substituted by 1 or 2 C₁₋₆ alkyl groups;
R₁ represents hydrogen, alkyl, alkenyl or cycloalkyl;
one or two of the group of R₂, R₃ and R₄ represents nitro the remaining members of the group of R₂, R₃ and R₄ represent hydrogen;
X represents a -CO-NH- moiety; and
Z represents C₂₋₄ n-alkylene group wherein each carbon is optionally substituted by 1 or 2 C₁₋₆ alkyl groups.

Example 2 of WO 96/13479 is the non-solvated hydrochloride salt, N-[3-[[2-(3,4-dimethoxyphenyl)ethyl]amino]propyl]-4-nitrobenzamide hydrochloride (hereinafter also referred to as 'the Hydrochloride'), the disclosed melting point of which is 141-2°C.

It has now been discovered that N-[3-[[2-(3,4-dimethoxyphenyl)ethyl]amino]propyl]-4-nitrobenzamide hydrochloride exists in a novel hydrated form which form is particularly suitable for bulk preparation and handling and is also indicated to have superior formulation properties. This novel hydrated form can be prepared by an efficient, economic and reproducible process particularly suited to large scale preparation.

The novel form also has useful pharmaceutical properties and is considered to be a useful anti-arrhythmic agent having combined Class III/Class IV anti-arrhythmic properties, therefore showing an improved pharmacological profile over pure class III anti-arrhythmic agents, in particular showing a low proarrhythmic potential, readily restoring the contractile function of the ischaemic myocardium. It is considered to be particularly useful for the treatment of atrial or ventricular cardiac arrhythmias.

Accordingly, the present invention provides hydrated N-[3-[[2-(3,4-dimethoxyphenyl)ethyl]amino]propyl]-4-nitro benzamide hydrochloride (hereinafter also referred to as 'Compound (I)') characterised in that it:
(i) comprises water in the range of from 1.7 to 2.4 molar equivalents; and/or
(ii) has a melting point above 145°C and
(iii) provides an infra red spectrum containing peaks at 3510, 3342, 3076, 1665, 1598, 1343, 1330, 1216 and 801 cm⁻¹; and
(iv) provides a solid state nuclear magnetic resonance spectrum containing chemical shifts substantially as represented in Table 1; and
(v) provides an X-ray powder refraction (XRPD) pattern substantially as represented in Table II.

Suitably, Compound (I) comprises from 1.8 to 2.3 or 1.9 to 2.1 molar equivalents of water, especially 2.0 molar equivalents.

Suitably, the melting point of Compound (I) is in the range of from 150°C to 154°C, for example 150°C, 151°C, 152°C, 153°C and 154°C.

In a further aspect Compound (I) provides an infra red spectrum containing peaks at 3510, 3342, 3307, 3076, 1665, 1632, 1598, 1548, 1520, 1343, 1330, 1310, 1267, 1240, 1216, 1162, 1147, 1119, 1105, 1048, 1036, 1025, 981, 921, 891, 873, 854, 801, 767, 720, 626, 573, 553 and 500 cm^{-1.}

Suitably, Compound (I) provides an infra red spectrum substantially as illustrated in Figure (I).

Suitably, Compound (I) provides a solid state nuclear magnetic resonance spectrum containing chemical shifts substantially as represented in Table I.

Suitably, Compound (I) provides an X-ray powder refraction (XRPD) pattern substantially as represented in Table II.

The present invention encompasses Compound (I) isolated in pure form or when admixed with other materials, for example the known anhydrous form of the Hydrochloride or any other material.

Preferably, Compound (I) is in a crystalline form.

The invention also provides a process for preparing the hydrated N-[3-[[2-(3,4-dimethoxyphenyl)ethyl]amino]propyl]-4-nitrobenzamide hydrochloride, characterised in that N-[3-[[2-(3,4-dimethoxyphenyl)ethyl]amino]propyl]-4-nitrobenzamide hydrochloride is hydrated in the presence of the required amount of water.

Suitable hydration methods include conventional hydration methods such as crystallisation, including recrystallisation, of the Hydrochloride from water or an aqueous solvent.

A suitable aqueous solvent is an aqueous organic solvent such as an aqueous alkanol, for example aqueous methanol, aqueous ethanol and aqueous propanol, or aqueous tetrahydrofuran or aqueous acetone, and mixtures thereof.

Suitable aqueous solvents contain up to 15% water by volume, preferably 2.5% to 10% by volume.

Crystallisation and any recrystallisation is generally carried out at low to ambient temperature, suitably at ambient temperature.

Preferably, the crystallisation is initiated by seeding with crystals of the hydrated form but this is not essential.

Conveniently, crystallisation is effected by allowing the aqueous solvent to cool from an elevated temperature, which temperature depends of course upon the nature of the solvent, an example is a temperature in the range of from 50°C to 100°C.

In a preferred form of the process, Compound (I) is prepared from a solution of the Hydrochloride in aqueous ethanol at an elevated temperature such as 60°C, allowing the product to crystallise on cooling and thereafter, if required, recrystallising the product from an appropriate aqueous solvent, usually aqueous ethanol. Purification of Compound (I) is also suitably effected by recrystallization of impure Compound (I) using this last mentioned procedure.

In an alternative hydration, the Hydrochloride is hydrated in an atmosphere of water vapour, at an ambient or, preferably, an elevated temperature, for example 40°C until Compound (I) is formed; conveniently hydration is continued until constant weight is achieved.

In a further hydration, N-[3-[[2-(3,4-dimethoxyphenyl)ethyl]amino]propyl]-4-nitrobenzamide hydrochloride is prepared *in -situ* in an aqueous solvent and then allowed to crystallise as described above.

The Hydrochloride is prepared according to known procedures such as those disclosed in WO 96/13479. The disclosures of WO 96/13479 are incorporated herein by reference.

As used herein 'aqueous solvent' includes single organic solvents or mixtures of organic solvents which contain sufficient water to provide product with 1.7 to 2.4 molar equivalents of water ('the required level' or 'the required amount' of water); usually, the level of water present is in excess of the required level.

As used herein, the term "cardiac arrhythmia" relates to any variation from the normal rhythm of heart beat, including, without limitation, sinus arrhythmia, premature heartbeat, heartblock, fibrillation, flutter, tachycardia, paroxysmal tachycardia and premature ventricular contractions.

As mentioned above the compound of the invention has useful therapeutic properties: The present invention accordingly provides Compound (I) for use as an active therapeutic substance.

More particularly, the present invention provides a Compound (I) for use in the treatment of and/or prophylaxis of arrhythmia, especially cardiac arrhythmia such as ventricular arrhythmia, and also ischaemic rhythm disorders.

Compound (I) may be administered per se or, preferably, as a pharmaceutical composition also comprising a pharmaceutically acceptable carrier.

Accordingly, the present invention also provides a pharmaceutical composition comprising Compound (I) and a pharmaceutically acceptable carrier therefor.

Compound (I) is normally administered in unit dosage form.

An amount effective to treat the disorder hereinbefore described depends upon such factors as the efficacy of a Compound (I) chosen, the nature and severity of the disorders being treated and the weight of the mammal. However, a unit dose will normally contain 0.1 to 500 mg for example 2 to 50 mg, of the compound of the invention. Unit doses will normally be administered once or more than once a day, for example 2,3,4,5 or 6 times a day, more usually 2 to 4 times a day, such that the total daily dose is normally in the range, for a 70 kg adult of 0.1 to 2500 mg, more usually 1 to 1000 mg, for example 1 to 200 mg, that is in the range of approximately 0.02 to 3 mg/kg/day, more usually 0.1 to 3 mg/kg/day, for example 0.15 to 2 mg/kg/day.

At the above described dosage range, no toxicological effects are indicated for the compounds of the invention.

In such treatment, the active compound may be administered by any suitable route, e.g. by the oral, parenteral or topical routes. For such use, the compound will normally be employed in the form of a pharmaceutical composition in association with a human or veterinary pharmaceutical carrier, diluent and/or excipient, although the exact form of the composition will naturally depend on the mode of administration.

Compositions are prepared by admixture and are suitably adapted for oral, parenteral or topical administration, and as such may be in the form of tablets, capsules, oral liquid preparations, powders, granules, lozenges, pastilles, reconstitutable powders, injectable and infusable solutions or suspensions, suppositories and transdermal devices. Orally administrable compositions are preferred, in particular shaped oral compositions, since they are more convenient for general use.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art.

Suitable fillers for use include cellulose, mannitol, lactose and other similar agents. Suitable disintegrants include starch, polyvinylpyrrolidone and starch derivatives such as sodium starch glycollate. Suitable lubricants include, for example, magnesium stearate. Suitable pharmaceutically acceptable wetting agents include sodium lauryl sulphate.

Solid oral compositions may be prepared by conventional methods of blending, filling, tabletting or the like. Repeated blending operations may be used to distribute the active agent throughout those compositions employing large quantities of fillers. Such operations are, of course, conventional in the art.

Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents.

For parenteral administration, fluid unit dose forms are prepared containing a compound of the present invention and a sterile vehicle. The compound, depending on the vehicle and the concentration, can be either suspended or dissolved. Parenteral solutions are normally prepared by dissolving the active compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the active compound is suspended in the vehicle instead of being dissolved and sterilised by exposure to ethylene oxide before suspending in the sterile vehicle. Advantageously, a surfactant or wetting agent is included in the composition to facilitate uniform distribution of the active compound.

For topical administration, the composition may be in the form of a transdermal ointment or patch for systemic delivery of the compound and may be prepared in a conventional manner, for example, as described in the standard textbooks such as 'Dermatological Formulations' - B.W. Barry (Drugs and the Pharmaceutical Sciences - Dekker) or Harrys Cosmeticology (Leonard Hill Books).

In addition such compositions may contain further active agents such as anti-hypertensive agents and diuretics.

As is common practice, the compositions will usually be accompanied by written or printed directions for use in the medical treatment concerned.

As used herein the term 'pharmaceutically acceptable' embraces compounds, compositions and ingredients for both human and veterinary use: for example the term 'pharmaceutically acceptable salt' embraces a veterinarily acceptable salt.

The present invention further provides a method for the treatment and/or prophylaxis of arrhythmia, especially cardiac arrhythmia such as ventricular arrhythmia, and also ischaemic rhythm disorders in a human or non-human mammal which comprises administering an effective, non-toxic, amount of Compound (I) to a human or non-human mammal in need thereof.

Conveniently, the active ingredient may be administered as a pharmaceutical composition hereinbefore defined, and this forms a particular aspect of the present invention.

In the treatment and/or prophylaxis of arrhythmia and/or ischaemic arrhythmia disorders Compound (I) may be taken in doses, such as those described above.

Similar dosage regimens are suitable for the treatment and/or prophylaxis of non-human mammals.

In a further aspect the present invention provides the use of Compound (I) for the manufacture of a medicament for the treatment of arrhythmia, especially cardiac arrhythmia such as ventricular arrhythmia, and also ischaemic rhythm disorders.

No adverse toxicological effects are indicated when Compound (I) is administered in the above mentioned dosage ranges.

The following Examples illustrate the invention but do not limit it in any way.

### Example 1

### Preparation of N-[3-[[2-(3,4-dimethoxyphenyl)ethyl]amino]propyl]-4-nitrobenzamide, hydrochloride hydrate

A solution of 9:1 ethanol:water (v/v) (7.5 litres) was added to N-[3-[[2-(3,4-dimethoxyphenyl)ethyl]amino]propyl]-4-nitrobenzamide hydrochloride (2.44 kg, 4.86 moles). The stirred suspension was then heated to 60°C to give a clear solution. This solution was hot filtered then cooled to 30°C in a water bath. A small sample was removed and scratched to induce crystallisation. The crystals were added to the bulk solution and this was allowed to stir and crystallise overnight at ambient temperature. The resulting suspension was cooled in an ice bath for 2 hrs. The solid product was filtered, washed with 9:1 ethanol:water (v/v) (1.5 litres), then ethanol (750 mls) and dried in a vacuum oven fitted with a filtered air bleed at 30-33°C to constant weight to give the titled product as a yellow solid.

### Example 2

### Preparation of N-[3-[[2-(3,4-dimethoxyphenyl)ethyl]amino]propyl]-4-nitrobenzamide, hydrochloride hydrate

An oven containing trays of cotton wool saturated with water was preheated to 40°C. N-(3((2-(3,4-dimethoxyphenyl)ethyl)amino)propyl)-4-nitrobenzamide hydrochloride (100g) was placed on a loosely covered tray in the oven and left to hydrate at 40°C. When the product had achieved constant weight it was removed from the oven and left open to the atmosphere to equilibrate to ambient temperature to give 109.1 g of the titled compound as a yellow solid

### Example 3

### Preparation of N-[3-[[2-(3,4-dimethoxyphenyl)ethyl]amino]propyl]-4-nitrobenzamide, hydrochloride hydrate

N-[3-[[2-(3,4-dimethoxyphenyl)ethyl]amino]propyl]-4-nitrobenzamide, hydrochloride (100g) was suspended in industrial methylated spirits (IMS) (300mls) and water(34mls).The mixture was heated to give a solution. The hot solution was cooled to ambient temperature in a water bath for 30 minutes. The resulting suspension was stirred at ambient temperature overnight then cooled in an ice-bath for 1.5hrs. The solid product was filtered and washed with IMS (100mls) and left open to the atmosphere to equilibrate at ambient temperature to give 104.2g of the titled product as a yellow solid.

### Example 4

### Preparation of N-[3-[[2-(3,4-dimethoxyphenyl)ethyl]amino]propyl]-4-nitrobenzamide, hydrochloride hydrate

A solution of N-[3-[[2-(3,4-dimethoxyphenyl)ethyl]amino]propyl]-4-nitrobenzamide (211g) in tetrahydrofuran (THF, 650mls) was stirred at ambient temperature. Concentrated hydrochloric acid (62mls) was added. The reaction temperature rose to 50°C. The mixture was cooled in an ice-bath to 25°C then stirred at ambient temperature overnight. The suspension was cooled in an ice-bath for 2hrs, the crystalline product filtered, washed with THF (250mls) and left open to the atmosphere to equilibrate at ambient temperature to give the titled compound as a yellow solid.

### SPECTROSCOPIC DATA - for N-[3-[[2-(3,4-dimethoxyphenyl)ethyl]amino]propyl]-4-nitrobenzamide, hydrochloride hydrate.

### (A) Solid State¹³C Nuclear Magnetic Resonance (NMR)

The 90.55MHz ¹³C CP-MAS NMR spectrum chemical shifts are tabulated in Table I. Samples were packed with minimal grinding into a 4 mm magic angle spinning (MAS) zirconia rotor fitted with a Kel-F cap, using sufficient material (ca. 50 mg) to fill the rotor just short of the cap space. No further sample preparation was necessary.

Spectra were run at ambient temperature on an AMX360 instrument at a MAS frequency of 10kHz. Spectra were acquired by cross-polarization (CP) from Hartmann-Hahn matched protons at a field of 50 kHz. The CP contact time was 1.6 ms, and repetition time was 15s. Protons were decoupled at a field of 80 kHz during acquisition by using a two-pulse phase modulated (TPPM) composite sequence (150° flip angle; phase alternation of 7°). Chemical shifts were externally referenced to the carboxylate signal of a glycine test sample at 176.4ppm relative to TMS, and are regarded as accurate to within +/- 0.5ppm.

**Table I**

| C¹³ Chemical shifts (ppm) | | | | | | | | |
|---|---|---|---|---|---|---|---|---|
| 28.8 | 32.0 | 38.1 | 49.9 | 52.3 | 56.0 | 56.8 | 109.9 | 111.2 |
| 123.6 | 128.8 | 129.8 | 131.7 | 139.3 | 147.0 | 149.5 | 166.2 | |

### (B) X-Ray Powder Diffraction (XRPD)

A summary of the XRPD angles characteristic of the Compound I is given in Table II A PW1710 X-ray powder diffractometer (Cu X-ray source) was used to generate the spectrum using the following acquisition conditions:

| | |
|---|---|
| Tube anode | Cu |
| Generator tension | 40 kV |
| Generator current | 30 mA |
| Start angle | 3.5 °2θ |
| End angle | 35.0 °2θ |
| Step size | 0.005 |
| Time per step | 0.25 s |

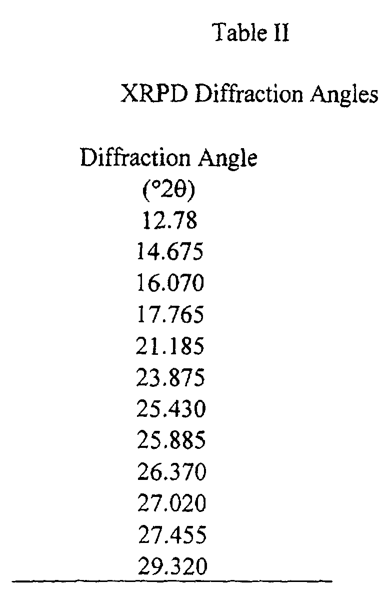

### (C) Infra Red spectrum

The infrared absorption spectrum of a mineral oil dispersion of compound (I) was obtained using a Perkin-Elmer 2000 FT-IR spectrometer at 2 cm⁻¹ resolution. Data were digitised at 0.5 cm⁻¹ intervals. The spectrum is shown in Figure (I).

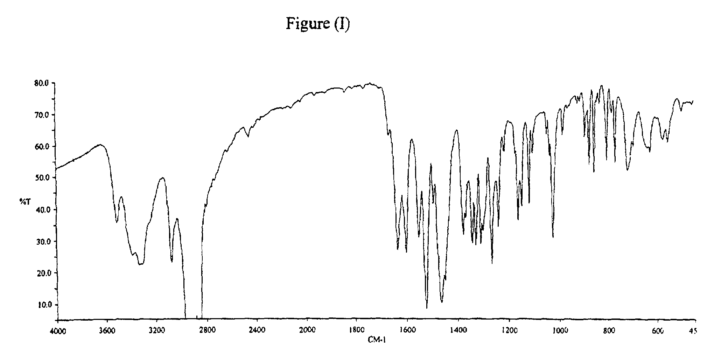

## Claims

1. Hydrated N-[3-[[2-(3,4-dimethoxyphenyl)ethyl]amino]propyl]-4-nitro benzamide hydrochloride **characterised in that** it:
(i) comprises water in the range of from 1.7 to 2.4 molar equivalents; and/or
(ii) has a melting point above 145°C and/or
(iii) provides an infra red spectrum containing peaks at 3510, 3342, 3076, 1665, 1598, 1343, 1330, 1216 and 801 cm⁻¹; and/or
(iv) provides a ¹³C solid state nuclear magnetic resonance spectrum containing chemical shifts at: 28.8, 32.0, 38.1, 49.9, 52.3, 56.0, 56.8, 109.9, 111.2 123.6, 128.8, 129.8, 131.7, 139.3, 147.0, 149.5, and 166.2 ppm; and/or
(v) provides an X-ray powder refraction (XRPD) pattern as follows:

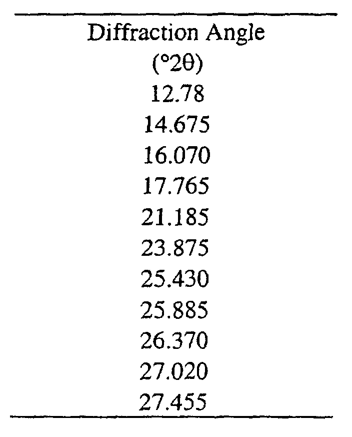

2. Hydrated N-[3-[[2-(3,4-dimethoxyphenyl)ethyl]amino]propyl]-4-nitro benzamide hydrochloride **characterised in that** it:
(i) comprises water in the range of from 1.7 to 2.4 molar equivalents; and
(ii) has a melting point above 145°C; and
(iii) provides an infra red spectrum containing peaks at 3510, 3342, 3076, 1665, 1598, 1343, 1330, 1216 and 801 cm⁻¹; and
(iv) provides a ¹³C solid state nuclear magnetic resonance spectrum containing chemical shifts at: 28.8, 32.0, 38.1, 49.9, 52.3, 56.0, 56.8, 109.9, 111.2, 123.6, 128.8, 129.8, 131.7, 139.3, 147.0, 149.5, and 166.2 ppm; and
(v) provides an X-ray powder refraction (XRPD) pattern as follows:

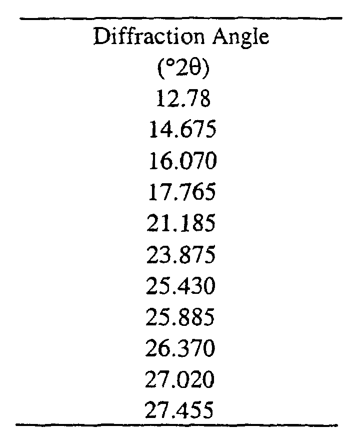

3. A compound according to claim 1 or claim 2, which comprises from 1.8 to 2.3 or 1.9 to 2.1 molar equivalents of water.

4. A compound according to any one of claims 1 to 3, which comprises 2.0 molar equivalents of water.

5. A compound according to any one of claims 1 to 4, which has a melting point in the range of from 150°C to 154°C.

6. A compound according to any one of claims 1 to 5, which has a melting point of 150°C, 151°C, 152°C, 153°C or 154°C.

7. A compound according to any one of claims 1 to 6, which provides an infra red spectrum containing peaks at 3510, 3342, 3307, 3076, 1665, 1632, 1598, 1548, 1520, 1343, 1330, 1310, 1267, 1240, 1216, 1162, 1147, 1119, 1105, 1048, 1036, 1025, 981, 921, 891, 873, 854, 801, 767, 720, 626, 573, 553 and 500 cm⁻¹.

8. A compound according to any one of claims 1 to 7, which provides an infra red spectrum substantially illustrated in figure (I):

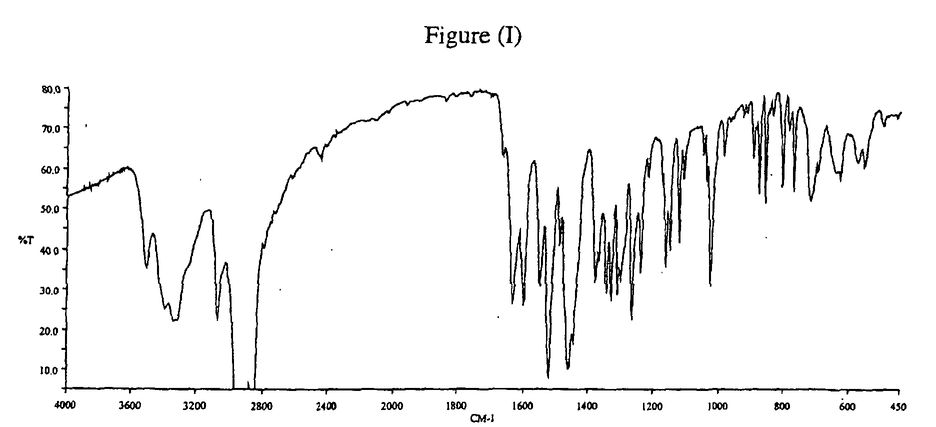

9. A process for preparing hydrated N-[3-[[2-(3,4-dimethoxyphenyl)ethyl]amino]propyl]-4-nitrobenzamide hydrochloride according to claim 1, **characterised in that** N-[3-[[2-(3,4-dimethoxyphenyl)ethyl]amino]propyl]-4-nitrobenzamide hydrochloride, is hydrated in the presence of the required amount of water.

10. A process according to claim 9, wherein the hydrochloride is crystallised or recrystallised from water or an aqueous solvent.

11. A pharmaceutical composition comprising compound (I) according to claim 1, or a pharmaceutically acceptable salt thereof and/or a pharmaceutically acceptable solvate thereof, and a pharmaceutically acceptable carrier.

12. Compound (I), according to claim 1, or a pharmaceutically acceptable salt thereof and/or a pharmaceutically acceptable solvate thereof, for use as an active therapeutic substance.

13. Compound (I), according to claim 1, or a pharmaceutically acceptable salt thereof and/or a pharmaceutically acceptable solvate thereof, for use in the treatment and/or prophylaxis of arrhythmia and ischaemic rhythm disorders.

14. The use of compound (I), according to claim 1, or a pharmaceutically acceptable salt thereof and/or a pharmaceutically acceptable solvate thereof, for the manufacture of a medicament for the treatment of arrhythmia and ischaemic rhythm disorders.

## Patentansprüche

1. Hydratisiertes N-[3-[[2-(3,4-Dimethoxyphenyl)ethyl]amino]propyl]-4-nitrobenzamid-Hydrochlorid, **dadurch gekennzeichnet, daß** es:
(i) Wasser im Bereich von 1,7 bis 2,4 Moläquivalenten umfaßt; und/oder
(ii) einen Schmelzpunkt oberhalb 145°C aufweist; und/oder
(iii) ein Infrarotspektrum ergibt, das Peaks bei 3510, 3342, 3076, 1665, 1598, 1343, 1330, 1216 und 801 cm⁻¹ enthält; und/oder
(iv) ein ¹³C-kernmagnetisches Resonanzspektrum in festem Zustand ergibt, das chemische Verschiebungen bei 28,8, 32,0, 38,1, 49,9, 52,3, 56,0, 56,8, 109,9, 111,2, 123,6, 128,8, 129,8, 131,7, 139,3, 147,0, 149,5 und 166,2 ppm enthält; und/oder
(v) ein Röntgen-Pulverbeugungsmuster (XRPD) ergibt, das wie folgt ist:
| Beugungswinkel (°2θ) |
|---|
| 12,78 |
| 14,675 |
| 16,070 |
| 17,765 |
| 21,185 |
| 23,875 |
| 25,430 |
| 25,885 |
| 26,370 |
| 27,020 |
| 27,455 |

2. Hydratisiertes N-[3-[[2-(3,4-Dimethoxyphenyl)ethyl]amino]propyl]-4-nitrobenzamid-Hydrochlorid, **dadurch gekennzeichnet, daß** es:
(i) Wasser im Bereich von 1,7 bis 2,4 Moläquivalenten umfaßt; und
(ii) einen Schmelzpunkt oberhalb 145°C aufweist; und
(iii) ein Infrarotspektrum ergibt, das Peaks bei 3510, 3342, 3076, 1665, 1598, 1343, 1330, 1216 und 801 cm⁻¹ enthält; und
(iv) ein ¹³C-kemmagnetisches Resonanzspektrum in festem Zustand ergibt, das chemische Verschiebungen bei 28,8, 32.0, 38,1, 49,9, 52,3, 56,0, 56,8, 109,9, 111,2, 123,6, 128,8, 129,8, 131,7, 139,3, 147,0, 149,5 und 166,2 ppm enthält; und
(v) ein Röntgen-Pulverbeugungsmuster (XRPD) ergibt, das wie folgt ist:
| Beugungswinkel (°2θ) |
|---|
| 12,78 |
| 14,675 |
| 16,070 |
| 17,765 |
| 21,185 |
| 23,875 |
| 25,430 |
| 25,885 |
| 26,370 |
| 27,020 |
| 27,455 |

3. Verbindung nach Anspruch 1 oder Anspruch 2, welche 1,8 bis 2,3 oder 1,9 bis 2,1 Moläquivalente Wasser umfaßt.

4. Verbindung nach einem der Ansprüche 1 bis 3, welche 2,0 Moläquivalente Wasser umfaßt.

5. Verbindung nach einem der Ansprüche 1 bis 4, welche einen Schmelzpunkt im Bereich von 150°C bis 154°C aufweist.

6. Verbindung nach einem der Ansprüche 1 bis 5, welche einen Schmelzpunkt von 150°C, 151°C, 152°C, 153°C oder 154°C aufweist.

7. Verbindung nach einem der Ansprüche 1 bis 6, welche ein Infrarotspektrum ergibt, das Peaks bei 3510, 3342, 3307, 3076, 1665, 1632, 1598, 1548, 1520, 1343, 1330, 1310, 1267, 1240, 1216, 1162, 1147, 1119, 1105, 1048, 1036, 1025, 981, 921, 891, 873, 854, 801, 767, 720, 626, 573, 553 und 500 cm⁻¹ enthält.

8. Verbindung nach einem der Ansprüche 1 bis 7, welche ein Infrarotspektrum ergibt, das im wesentlichen in Figur (I) dargestellt ist:

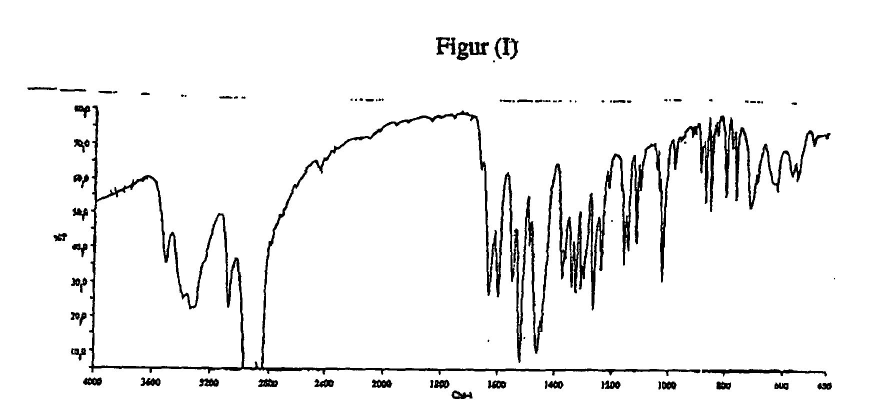

9. Verfahren zur Herstellung von hydratisiertem N-[3-[[2-(3,4-Dimethoxyphenyl)ethyl]amino]propyl]-4-nitrobenzamid-Hydrochlorid nach Anspruch 1, **dadurch gekennzeichnet, daß** N-[3-[[2-(3,4-Dimethoxyphenyl)ethyl]amino]propyl]-4-nitrobenzamid-Hydrochlorid in Gegenwart der erforderlichen Menge an Wasser hydratisiert wird.

10. Verfahren nach Anspruch 9, wobei das Hydrochlorid aus Wasser oder einem wäßrigen Lösungsmittel kristallisiert oder umkristallisiert wird.

11. Arzneimittel umfassend Verbindung (I) nach Anspruch 1 oder ein pharmazeutisch verträgliches Salz davon und/oder ein pharmazeutisch verträgliches Solvat davon und einen pharmazeutisch verträglichen Träger.

12. Verbindung (I) nach Anspruch 1 oder ein pharmazeutisch verträgliches Salz davon und/oder ein pharmazeutisch verträgliches Solvat davon zur Verwendung als therapeutischer Wirkstoff.

13. Verbindung (I) nach Anspruch 1 oder ein pharmazeutisch verträgliches Salz davon und/oder ein pharmazeutisch verträgliches Solvat davon zur Verwendung bei der Behandlung und/oder Prophylaxe von Arrhythmie und ischämischen Herzrhythmusstörungen.

14. Verwendung von Verbindung (I) nach Anspruch 1 oder eines pharmazeutisch verträglichen Salzes davon und/oder eines pharmazeutisch verträglichen Solvats davon zur Herstellung eines Medikaments zur Behandlung von Arrhythmie und ischämischen Herzrhythmusstörungen.

## Revendications

1. Chlorhydrate de N-[3-[[2-(3,4-diméthoxyphényl)-éthyl]amino]propyl]-4-nitrobenzamide hydraté, **caractérisé en ce que** :
(i) il comprend de l'eau en une quantité comprise dans l'intervalle de 1,7 à 2,4 équivalents molaires ; et/ou
(ii) il a un point de fusion supérieur à 145°C ; et/ou
(iii) il présente un spectre infrarouge contenant des pics à 3510, 3342, 3076, 1665, 1598, 1343, 1330, 1216 et 801 cm⁻¹ ; et/ou
(iv) il présente un spectre de résonance magnétique nucléaire de ¹³C à l'état solide contenant des déplacements chimiques à : 28,6, 32,0, 38,1, 49,9, 52,3, 56,0, 56,8, 109,9, 111,2, 123,6, 128,8, 129,8, 131,7, 139,3, 147,0, 149,5 et 166,2 ppm ; et/ou
(v) il présente le diagramme de diffraction des rayons X sur poudre (XRPD) suivant :
| Angle de diffraction (°2θ) |
|---|
| 12,78 |
| 14,675 |
| 16,070 |
| 17,765 |
| 21,185 |
| 23,875 |
| 25,430 |
| 25,885 |
| 26,370 |
| 27,020 |
| 27,455 |

2. Chlorhydrate de N-[3-[[2-(3,4-diméthoxyphényl)-éthyl]amino]propyl]-4-nitrobenzamide hydraté, **caractérisé en ce que** :
(i) il comprend de l'eau en une quantité comprise dans l'intervalle de 1,7 à 2,4 équivalents molaires ; et
(ii) il a un point de fusion supérieur à 145°C ; et
(iii) il présente un spectre infrarouge contenant des pics à 3510, 3342, 3076, 1665, 1598, 1343, 1330, 1216 et 801 cm⁻¹ ; et
(iv) il présente un spectre de résonance magnétique nucléaire de ¹³C à l'état solide contenant des déplacements chimiques à : 28,8, 32,0, 38,1, 49,9, 52,3, 56,0, 56,8, 109,9, 111,2, 123,6, 128,8, 129,8, 131,7, 139,3, 147,0, 149,5 et 166,2 ppm ; et
(v) il présente le diagramme de diffraction des rayons X sur poudre (XRPD) suivant :
| Angle de diffraction (°2θ) |
|---|
| 12,78 |
| 14,675 |
| 16,070 |
| 17,765 |
| 21,185 |
| 23,875 |
| 25,430 |
| 25,885 |
| 26,370 |
| 27,020 |
| 27,455 |

3. Composé suivant la revendication 1 ou la revendication 2, qui comprend 1,8 à 2,3 ou bien 1,9 à 2,1 équivalents molaires d'eau.

4. Composé suivant l'une quelconque des revendications 1 à 3, qui comprend 2,0 équivalents molaires d'eau.

5. Composé suivant l'une quelconque des revendications 1 à 4, qui a un point de fusion compris dans l'intervalle de 150°C à 154°C.

6. Composé suivant l'une quelconque des revendications 1 à 5, qui a un point de fusion de 150°C, 151°C, 152°C, 153°C ou 154°C.

7. Composé suivant l'une quelconque des revendications 1 à 6, qui présente un spectre infrarouge contenant des pics à 3510, 3342, 3307, 3076, 1665, 1632, 1598, 1548, 1520, 1343, 1330, 1310, 1267, 1240, 1216, 1162, 1147, 1119, 1105, 1048, 1036, 1025, 981, 921, 891, 873, 854, 801, 767, 720, 626, 573, 553 et 500 cm⁻¹.

8. Composé suivant l'une quelconque des revendications 1 à 7, qui présente un spectre infrarouge illustré essentiellement sur la figure (I) :

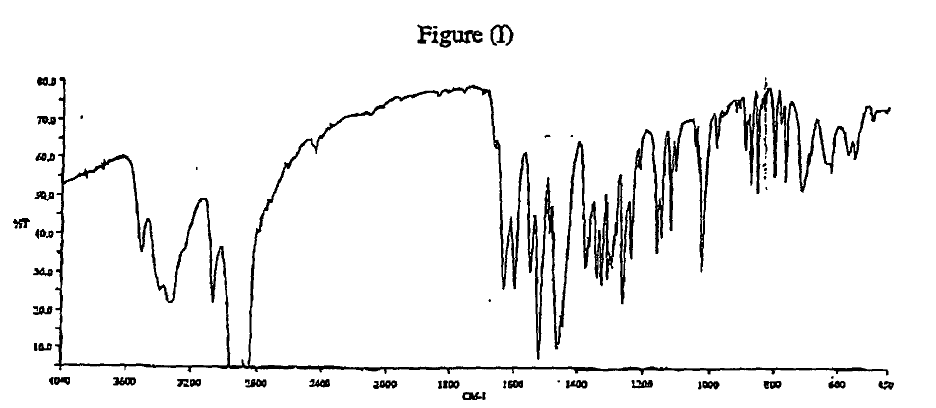

9. Procédé pour la préparation de chlorhydrate de N-[3-[[2-(3,4-diméthoxyphényl)éthyl] amino]propyl]-4-nitro-benzamide hydraté suivant la revendication 1, **caractérisé en ce que** le chlorhydrate de N-[3-[[2-(3,4-diméthoxyphényl)éthyl]amino]propyl]-4-nitro-benzamide est hydraté en présence de la quantité requise d'eau.

10. Procédé suivant la revendication 9, dans lequel le chlorhydrate est cristallisé ou recristallisé dans l'eau ou un solvant aqueux.

11. Composition pharmaceutique comprenant le composé (I) suivant la revendication 1, ou un de ses sels pharmaceutiquement acceptables et/ou un de ses produits de solvatation pharmaceutiquement acceptables, et un support pharmaceutiquement acceptable.

12. Composé (I) suivant la revendication 1, ou un de ses sels pharmaceutiquement acceptables et/ou un de ses produits de solvatation pharmaceutiquement acceptables, destinés à être utilisés comme substance thérapeutique active.

13. Composé (I) suivant la revendication 1, ou un de ses sels pharmaceutiquement acceptables et/ou un de ses produits de solvatation pharmaceutiquement acceptables, destinés à être utilisés dans le traitement et/ou la prophylaxie de l'arythmie et de troubles du rythme ischémiques.

14. Utilisation du composé (I) suivant la revendication 1, ou d'un de ses sels pharmaceutiquement acceptables et/ou d'un de ses produits de solvatation pharmaceutiquement acceptables pour la production d'un médicament destiné au traitement de l'arythmie et de troubles du rythme ischémiques.
